# EUROPEAN PATENT SPECIFICATION

(11) **EP 0 549 250 B1**
(45) Date of publication and mention of the grant of the patent: **27.09.1995**
(21) Application number: 92311488.8
(22) Date of filing: 16.12.1992
(51) Int. Cl.: C07C 69/716, C07C 67/347

(54) **Process for preparing 3-(2-oxocyclohexyl)propionic ester**
Verfahren zur Herstellung eines 3-(2-Oxocyclohexyl)propionsäureesters
Procédé de préparation d'un ester 3-(2-cétocyclohexyl)propionique

(30) Priority: 20.12.1991 JP 338239/91
(43) Date of publication of application: 30.06.1993
(73) Proprietor: SUMITOMO CHEMICAL COMPANY LIMITED, Osaka-shi, Osaka 541 (JP)
(72) Inventor: Sakai, Kiyomi, Niihama-shi, Ehime (JP); Nishida, Yoshitaka, Niihama-shi, Ehime (JP); Shirafuji, Tamio, Niihama-shi, Ehime (JP)
(74) Representative: Dixon, Donald Cossar

(56) References cited:
- CHEMICAL ABSTRACTS, vol. 115, no. 19, 11 November 1991, Columbus, Ohio, US; abstract no. 207573, page 979 ;column 2 ;
- CHEMICAL ABSTRACTS, vol. 109, no. 21, 21 November 1988, Columbus, Ohio, US; abstract no. 189888, page 664 ;column 1 ;

## Description

The present invention relates to a process for preparing a 3-(2-oxocyclohexyl)propionic ester from cyclohexanone or a derivative thereof and an acrylic ester using an amine as a catalyst. 3-(2-Oxocyclohexyl)propionic esters are important intermediates for producing 3,4-dihydrocoumarins or coumarin useful as perfumes or intermediates of dyes, agricultural chemicals or pharmaceuticals.

It has been proposed that addition of an acrylic acid ester, i.e., an α,β-unsaturated carboxylic acid ester, to cyclohexanone or a derivative thereof having active hydrogen at the α-position thereof yields a 3-(2-oxocyclohexyl)propionic ester, as described, e.g., in Journal of American Chemical Society, Vol. 85, pp. 207-222 (1968), JP-A-55-28983 and JP-A-58-79956. The term "JP-A" as used herein means an "unexamined published Japanese patent application. According to any of the known techniques, the addition reaction is effected by heating in the presence of an amine catalyst in a closed reactor or a reflux reactor without taking any starting materials or any reaction products out of the reaction system during the reaction.

Modifications which have so far been made to the above-mentioned process include driving water present in the reaction system out of the system by distillation, as disclosed in JP-A-62-258341 and adding the acrylic ester dropwise, as disclosed in JP-A-3-109353.

However, the reaction rate attained by the conventional processes is not sufficiently fast. Even where the reaction is carried out while removing water from the reaction system by azeotropic distillation, the reaction rate is still unsatisfactory.

An object of the present invention is to provide an improvement in the process for preparing a 3-(2-oxocyclohexyl)propionic ester from cyclohexanone or a derivative thereof and an acrylic ester, which attains an increased reaction rate.

The present inventors have conducted extensive investigations on the reaction between cyclohexanone and an acrylic ester to produce a 3-(2-oxocyclohexyl)propionic acid ester. As a result, it has now been found that the reaction rate is influenced by the amount of water in the reaction system, and water in the reaction system cannot be sufficiently removed by azeotropic distillation during the reaction when the reaction system contains a large quantity of an acrylic ester. It has been found that the reaction rate may be increased by adjusting the water content in the reaction system prior to commencement of the reaction.

The present invention provides a process for preparing a 3-(2-oxocyclohexyl)propionic ester represented by formula (III): 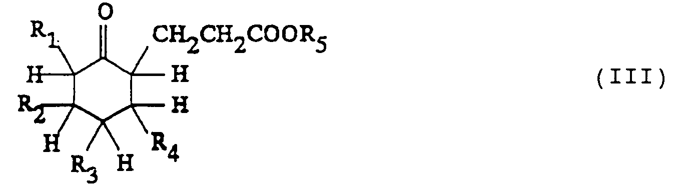
wherein R₁, R₂, R₃, and R₄ each represent a hydrogen atom or an alkyl group having from 1 to 5 carbon atoms; and R₅ represents an alkyl group having from 1 to 5 carbon atoms,
said process comprising the step of reacting cyclohexanone or a derivative thereof represented by formula (I): 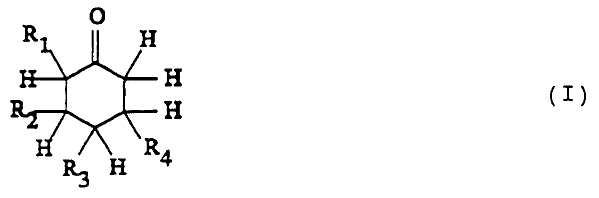
wherein R₁, R₂, R₃, and R₄ are as defined above,
with an acrylic ester represented by formula (II):

CH₂=CHCOOR₅ (II)

wherein R₅ is as defined above,
in the presence of an amine catalyst,
the water content of the reaction system being adjusted to 0.1 to 1.4 mols per mole of the amine catalyst before commencement of the reaction.

The derivatives of cyclohexanone which can be used in the present invention and not particularly limited, and examples thereof include 2-methylcyclohexanone, 4-methylcyclohexanone, 2-ethylcyclohexanone, 3-ethylcyclohexanone, 4-propylcyclohexanone, 4-butylcyclohexanone, 4-pentylcyclohexanone, 4-t-butylcyclohexanone, 2,4-dimethylcyclohexanone, 2-ethyl-4-methylcyclohexanone, 2,3,4-trimethylcyclohexanone, and 2,3,4,5-tetraethylcyclohexanone.

The acrylic ester which can be used in the present invention is not particularly limited, and examples thereof include methyl acrylate, ethyl acrylate, propyl acrylate, butyl acrylate, pentyl acrylate, and isopropyl acrylate.

While the molar ratio of the cyclohexanone or a derivative thereof (hereinafter inclusively referred to as "cyclohexanone derivative") to the acrylic ester is stoichiometrically 1/1, side reactions may be suppressed by using the cyclohexanone derivative in excess. The optimal molar ratio of the cyclohexanone derivative to the acrylic ester can be experimentally determined easily and is preferably from 1.2 to 2.

If desired, in order to prevent polymerization of the acrylic ester during the reaction, a polymerization inhibitor, such as hydroquinone, may be added to the reaction system.

Examples of the amine catalyst which can be used in the present invention include primary amines, secondary amines, tertiary amines, ammonia, and so on, with secondary amines being preferred. Pyrrolidine is the most preferred. A commercially available amine may be used as such.

The amine catalyst can be merely added as such to the reaction system. There is no need to previously form an addition product between the amine and the cyclohexanone derivative or acrylic ester. The catalyst is preferably used in an amount of about from 0.01 to 0.2 mol per mol of the acrylic ester. If the catalyst amount is too small, the reaction rate is reduced. If it is too large, the reaction may proceed at an increased rate but tends to be accompanied with considerable side reactions, resulting in a reduction in yield of the desired compound.

The reaction can be carried out by a method in which a cyclohexanone derivative, an acrylic ester, an amine, and, if desired, a solvent, etc. are charged all at once, followed by heating to a prescribed temperature, or alternatively, a method in which a cyclohexanone derivative, an amine, and, if desired, a solvent, etc. are first charged and, after heating to a prescribed temperature, an acrylic ester is added thereto dropwise. The latter mode is usually preferred.

The reaction is generally conducted at a temperature of about from 50 to 220°C, and preferably about from 100 to 200°C. If the reaction temperature exceeds about 220°C, the yield of the 3-(2-oxocyclohexyl)propionic acid ester tends to decrease. If it is lower than about 50°C, the reaction rate tends to be reduced too much. The reaction pressure is not critical and is appropriately controlled so that the reaction may proceed in a liquid phase.

While hydrocarbons may be used as a solvent as is conventionally known, use of the cyclohexanone derivative in excess with respect to the acrylic ester can eliminate the need of addition of any solvent.

The apparatus for carrying out the reaction is not particularly limited, and any of those having a stirring mechanism can be used. For example, a mixing vessel equipped with a simple stirring blade or a vessel equipped with an external circulating pump may be employed. The reaction system may be either continuous or batchwise.

In the reaction, while it is feasible to convert either the whole or a part of the acrylic ester, it is preferred to convert at least 90% of the acrylic ester. In order to reduce the amount of the circulating unreacted acrylic ester which exhibits relatively high polymerizability, it is desirable to convert at least 99% of the acrylic ester. After completion of the reaction, the reaction mixture is separated, for example, by distillation, and the unreacted materials, i.e., the cyclohexanone derivative and/or acrylic ester, can be recovered and reused.

In the process of the present invention, the water content of the reaction system must be adjusted to 0.1 to 1.4 mols per mol of the amine catalyst before commencement of the reaction. The water content of the reaction system before the reaction includes water content produced in the system before the reaction and water content brought into the system. The former consists primarily of the water content resulting from condensation upon mixing of a cyclohexanone derivative and an amine, and secondarily of the water content resulting from dimerization of a trace amount of the cyclohexanone derivative. The latter is water brought in with the reactants (i.e., cyclohexanone or acrylic ester), solvent, and the like, and mostly that brought in with a recovered cyclohexanone derivative for the following reason.

Since the cyclohexanone derivative is preferably used in excess, the excess cyclohexanone derivative remaining unreacted after the reaction is recovered by distillation for reuse. The thus recovered cyclohexanone derivative usually contains from about 0.1 to 1% by weight of water, which would be brought into the reaction system upon reusing. Although the mechanism of water production accompanying distillation of the cyclohexanone derivative has not yet been clarified, it is considered that the distillation is accompanied by condensation of the amine and the produced 3-(2-oxocyclohexyl)propionic acid ester to form an enamine and water, which enters the recovered cyclohexanone derivative and is then brought into the reaction system.

Adjustment of the water content in the reaction system can be effected as follows: The water content of each material to be charged, i.e., the cyclohexanone derivative, acrylic ester, amine, solvent, etc., may be adjusted. Adjustment of the water content in each material can be effected in any known manner. Of these materials, fresh ones do not need to be subjected to the adjustment as long as their water content is as small as that usually contained in a commercial product. However, it is preferable that the recovered cyclohexanone derivative should undergo the water content adjustment, because it usually has a water content of from about 0.1 to 1% by weight as mentioned above.

The water content can be removed by, for example, azeotropic distillation, a treatment with a dehydrating agent, bubbling with an inert gas, and the like. In general, the recovered cyclohexanone derivative is used after being dehydrated by azeotropic distillation. Alternatively, the recovered cyclohexanone derivative is charged along with the amine, and then the mixture of the cyclohexanone derivative and the amine is dehydrated by azeotropic distillation. Where water is separated by means of a water-separator during the reaction, it is preferably driven out of the system. If the water content in the reaction system becomes too small, the reaction hardly proceeds. This being the case, water is externally supplied according to necessity.

In the case where a cyclohexanone derivative, an acrylic ester, an amine, and, if desired, a solvent are charged all at once, or where a cyclohexanone derivative and an amine are first charged and then the reaction is conducted while adding an acrylic ester thereto, the water content in the system before commencement of the reaction can be expressed as the sum of (a) and (b): (a) The total water content present in the cyclohexanone derivative, acrylic ester, amine, solvent, etc. (b) The amount of water equimolar with the amine.

In the case where a cyclohexanone derivative and an amine are first charged and subjected to a dehydration operation before addition of an acrylic ester, the water content in the system before commencement of the reaction can be expressed as the sum of (c1) and (d) or the sum of (c2) and (d): (c1) The water content obtained by subtracting the water content removed by the dehydration operation from the above-mentioned water content of the sum of (a) and (b). (c2) The water content determined after the dehydration operation. (d) The water content of the acrylic ester.

The method of water content determination is not particularly limited. For example, Karl Fischer's method using acetic acid, etc. or azeotropic distillation can be employed.

Although it is not clear how the reaction rate depends on the water content in the reaction mixture, the following assumption could be made safely. A cyclohexanone derivative and an amine react to produce an enamine of cyclohxanone derivative and, as a by-product, water. The thus formed enamine then reacts with an acrylic ester to form an enamine of a 3-(2-oxocyclohexyl)propionic ester. This enamine reacts with water in the system to form a 3-(2-oxocyclohexyl)propionic ester to regenerate the amine. With an increasing water content in the reaction mixture, the proportion of the amine which is not in an enamine form, but in its amine form, increases and undergoes reaction with an acrylic ester to form an adduct. The amine in the form of the adduct has a lower catalytic activity. On the other hand, if the water content in the reaction mixture is too small, the above-mentioned course of reaction in which the enamine of a 3-(2-oxocyclohexyl)propionic ester reacts with water to become a 3-(2-oxocyclohexyl)propionic ester hardly proceeds, thus resulting in retardation of the total reaction.

The present invention thus makes it possible to produce a 3-(2-oxocyclohexyl)propionic acid ester at a sufficiently high reaction rate.

The present invention is now illustrated in greater detail with reference to Examples, but it should be understood that the present invention is not construed as being limited thereto. All the percents are by weight unless otherwise indicated.

### EXAMPLE 1

In a 1 ℓ round flask fitted with a separator of distilled water were charged 156 g of recovered cyclohexanone having a water content of 0.464%, 290 g of cyclohexanone having a water content of 0.04%, and 6.5 g of pyrrolidine. The content of the flask was refluxed at 160°C for 30 minutes for dehydration to a water content of 0.14%, which corresponded to 0.37 mol per mol of pyrrolidine. The temperature was lowered to 110°C, and 254 g of methyl acrylate was added dropwise thereto at a feed rate of 1.7 mℓ/min to conduct a reaction while maintaining at 150°C. The time required for the conversion of methyl acrylate to exceed 99 mol% was 6 hours inclusive of the time for dropwise addition of methyl acrylate.

After completion of the reaction, the reaction mixture was analyzed by gas chromatography. It was found that the conversion of methyl acrylate was 99.8 mol%, and the yield and selectivity of methyl 3-(2-oxocyclohexyl)propionate were 93.5 mol% and 93.7 mol%, respectively, based on methyl acrylate.

### COMPARATIVE EXAMPLE 1

In a 1 ℓ round flask fitted with a separator of distilled water were charged 156 g of recovered cyclohexanone having a water content of 0.464%, 290 g of cyclohexanone having a water content of 0.04%, and 6.5 g of pyrrolidine. The water content in the mixture was 1.49 mols per mol of pyrrolidine. The temperature was elevated to 110°C, and 254 g of methyl acrylate was added thereto at a feed rate of 1.7 mℓ/min to conduct a reaction while maintaining at 150°C. The water separated by the water-separator was withdrawn. The time required for the conversion of methyl acrylate to exceed 99 mol% was 13 hours inclusive of the time for dropwise addition of methyl acrylate.

### EXAMPLE 2

In a 2 ℓ round flask fitted with a separator of distilled water were charged 886 g of cyclohexanone having a water content of 0.04% and 12.9 g of pyrrolidine. The water content in the mixture was 1.11 mols per mol of pyrrolidine. The temperature was elevated to 110°C, and 518 g of methyl acrylate was added dropwise thereto at a feed rate of 3.5 mℓ/min to conduct a reaction while maintaining at 150°C. The time required for the conversion of methyl acrylate to exceed 99 mol% was 7 hours inclusive of the time for dropwise addition of methyl acrylate.

After completion of the reaction, the reaction mixture was analyzed by gas chromatography. It was found that the conversion of methyl acrylate was 99.9 mol%, and the yield and selectivity of methyl 3-(2-oxocyclohexyl)propionate were 95.1 mol% and 95.2 mol%, respectively, based on methyl acrylate.

### COMPARATIVE EXAMPLE 2

In a 1 ℓ round flask fitted with a separator of distilled water were charged 155 g of recovered cyclohexanone having a water content of 0.464%, 290 g of cyclohexanone having a water content of 0.04%, and 6.4 g of pyrrolidine. The content of the flask was refluxed at 50 Torr for 30 minutes for dehydration to a water content of 0.023%, which corresponded to 0.06 mol per mol of pyrrolidine. The temperature was elevated to 110°C, and 263 g of methyl acrylate was added dropwise thereto at a feed rate of 1.7 mℓ/min to conduct a reaction while maintaining at 150°C. The time required for the conversion of methyl acrylate to exceed 99 mol% was 11 hours inclusive of the time for dropwise addition of methyl acrylate.

## Claims

1. A process for preparing a 3-(2-oxocyclohexyl)propionic ester represented by the general formula (III): 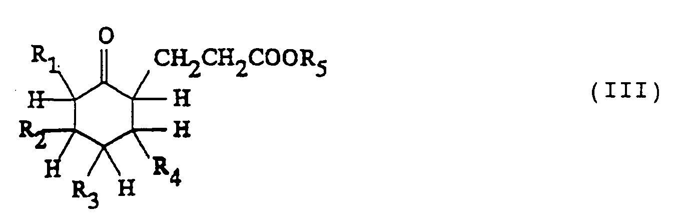 wherein R₁, R₂, R₃, and R₄ each represent a hydrogen atom or an alkyl group having from 1 to 5 carbon atoms; and R₅ represents an alkyl group having from 1 to 5 carbon atoms,
said process comprising the step of reacting a cyclohexanone or a derivative thereof represented by formula (I): 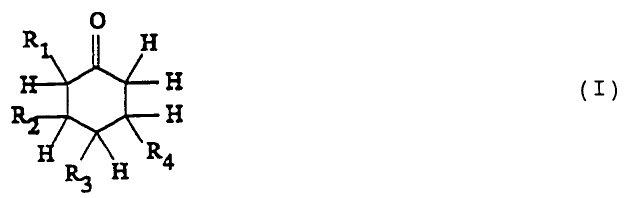 wherein R₁, R₂, R₃, and R₄ are as defined above,
with an acrylic ester represented by formula (II):
CH₂=CHCOOR₅ (II)
wherein R₅ is as defined above, in the presence of an amine catalyst, characterised in that the water content of the reaction system is adjusted to 0.1 to 1.4 moles per mole of the amine catalyst before commencement of the reaction.

2. A process as claimed in Claim 1, wherein the molar ratio of said cyclohexanone or derivative thereof to said acrylic ester is from 1.2 to 2.

3. A process as claimed in Claim 1 or 2, wherein said amine ctalyst is present in an amount of from 0.01 to 0.2 moles per mole of said acrylic ester.

4. A process as claimed in Claim 1, 2 or 3, wherein the reaction is carried out at a temperature of from 100 to 200°C.

5. A process as claimed in any preceding claim, wherein said acrylic ester is methyl acrylate.

6. A process as claimed in any preceding claim, wherein said cyclohexanone or derivative thereof is cyclohexanone.

7. A process as claimed in any preceding claim, wherein said amine catalyst is pyrrolidine.

## Patentansprüche

1. Verfahren zur Herstellung eines 3-(2-Oxocyclohexyl)propionsäureesters der allgemeinen Formel (III) 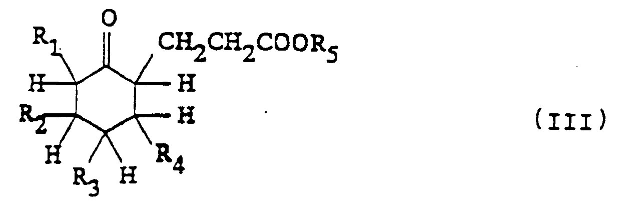 wobei R₁, R₂, R₃ und R₄ jeweils ein Wasserstoffatom oder einen Alkylrest mit 1 bis 5 Kohlenstoffatomen darstellen und R₅ für einen Alkylrest mit 1 bis 5 Kohlenstoffatomen steht,
umfassend den Umsetzungsschritt von Cyclohexanon oder einem Derivat davon der Formel (I) 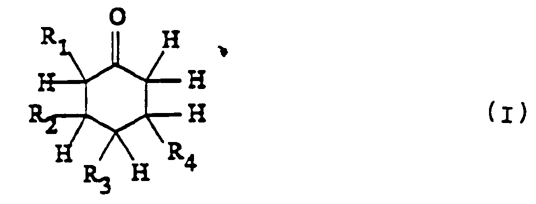 wobei R₁, R₂, R₃ und R₄ wie vorstehend definiert sind,
mit einem Acrylester der Formel (II)
CH₂=CH-COOR₅ (II)
wobei R₅ wie vorstehend definiert ist,
in Gegenwart eines Aminkatalysators, dadurch gekennzeichnet, daß der Wassergehalt im Reaktionssystem vor dem Beginn der Umsetzung auf 0,1 bis 1,4 Mol je Mol des Aminkatalysators eingestellt wird.

2. Verfahren nach Anspruch 1, wobei das Molverhältnis des Cyclohexanons oder eines Derivats davon zum Acrylester 1,2 bis 2 beträgt.

3. Verfahren nach Anspruch 1 oder 2, wobei der Aminkatalysator in einer Menge von 0,01 bis 0,2 Mol je Mol des Acrylesters vorhanden ist.

4. Verfahren nach Anspruch 1, 2 oder 3, wobei die Umsetzung bei einer Temperatur von 100 bis 200°C durchgeführt wird.

5. Verfahren nach einem der vorstehenden Ansprüche, wobei der Acrylester Methylacrylat darstellt.

6. Verfahren nach einem der vorstehenden Ansprüche, wobei das Cyclohexanon oder das Derivat davon Cyclohexanon darstellt.

7. Verfahren nach einem der vorstehenden Ansprüche, wobei der Aminkatalysator Pyrrolidin darstellt.

## Revendications

1. Procédé pour la préparation d'un ester 3-(2-oxocyclohexyl)propionique représenté par la formule générale 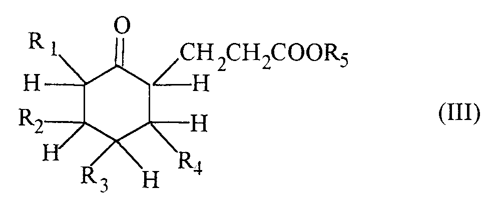 dans laquelle R₁, R₂, R₃ et R₄ représentent chacun un atome d'hydrogène ou un groupe alkyle ayant 1 à 5 atomes de carbone, et R₅ représente un groupe alkyle ayant 1 à 5 atomes de carbone,
ledit procédé comprenant l'étape de réaction de la cyclohexanone ou d'un de ses dérivés représentés par la formule (I) : 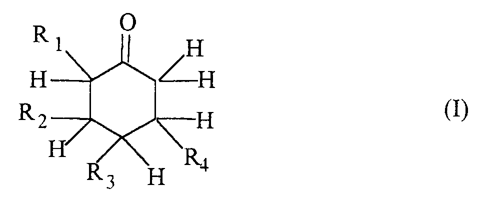 dans laquelle R₁, R₂, R₃ et R₄ sont tels que définis ci-dessus, avec un ester acrylique représenté par la formule (II) :
CH₂=CH COOR₅ (II)
dans laquelle R₅ est tel que défini ci-dessus, en présence d'un catalyseur aminé, caractérisé en ce que la quantité d'eau contenue dans le système réactionnel est ajustée à 0,1 à 1,4 mole par mole de catalyseur aminé avant le début de la réaction.

2. Procédé selon la revendication 1, dans lequel le rapport molaire de ladite cyclohexanone ou de son dérivé audit ester acrylique est compris entre 1,2 et 2.

3. Procédé selon une quelconque des revendications 1 ou 2, dans lequel le catalyseur aminé est présent en une quantité située entre 0,01 et 0,2 mole par mole dudit ester acrylique.

4. Procédé selon la revendication 1, 2 ou 3 dans lequel la réaction est conduite à une température située entre 100 et 200°C.

5. Procédé selon une quelconque des revendications qui précèdent, dans lequel ledit ester acrylique est l'acrylate de méthyle.

6. Procédé selon une quelconque des revendications qui précèdent, dans lequel ladite cyclohexanone ou son dérivé est la cyclohexanone.

7. Procédé selon une quelconque des revendications qui précèdent, dans lequel ledit catalyseur aminé est la pyrrolidine.
